**Europäisches Patentamt**

**European Patent Office**

**Office européen des brevets**

(11) Veröffentlichungsnummer: **0 142 623**

**A1**

(12) **EUROPÄISCHE PATENTANMELDUNG**

(21) Anmeldenummer: **84109478.2**

(22) Anmeldetag: **09.08.84**

(51) Int. Cl.⁴: **A 61 L 2/18**
**G 02 C 13/00**

(30) Priorität: **19.08.83 DE 3329922**

(43) Veröffentlichungstag der Anmeldung:
**29.05.85 Patentblatt 85/22**

(84) Benannte Vertragsstaaten:
**AT BE CH DE FR GB IT LI NL SE**

(71) Anmelder: **Firma Carl Zeiss**

**D-7920 Heidenheim (Brenz)(DE)**

(84) Benannte Vertragsstaaten:
**AT BE CH DE FR GB IT LI NL SE**

(71) Anmelder: **CARL-ZEISS-STIFTUNG Trading as CARL ZEISS**

**D-7920 Heidenheim (Brenz)(DE)**

(84) Benannte Vertragsstaaten:
**GB**

(72) Erfinder: **Polzhofer, Kurt, Dr.**
**Bellmannstrasse 8**
**D-2000 Hamburg 52(DE)**

(54) **Reinigungs- und Desinfektionsystem für harte und weiche Kontaktlinsen.**

(57) Reinigungs- und Desinfektionssystem für harte und weiche Kontaktlinsen bei dem abgelagerte Substanzen oxidativ zersetzt werden. Es finden zwei verschiedene Lösungen Verwendung, die jeweils frisch durch Einbringen einer Tablette in ein vorbestimmtes Volumen einer konservierten gepufferten Kochsalzlösung hergestellt werden. Die Tablette für die erste Lösung besteht aus Harnstoffperoxohydrat, die Tablette für die zweite Lösung besteht aus Natriumascorbat oder aus einer Mischung von L (+) Ascorbinsäure und Natriumbicarbonat.

0142623
83025x P EP

Reinigungs- und Desinfektionssystem für harte und weiche Kontaktlinsen

Die vorliegende Erfindung betrifft ein Reinigungs- und Desinfektionssystem für harte und weiche Kontaktlinsen, sowie ein Verfahren zum Reinigen und Desinfizieren mit diesem System.

Kontaktlinsen werden direkt auf der Hornhaut des Auges getragen. Die Bindehautflüssigkeit des Auges enthält neben anorganischen Salzen auch zahlreiche organische Stoffe wie Proteine und Lipide. Diese Stoffe lagern sich beim längeren Tragen auf der Kontaktlinse ab, wobei die sich bildenden Niederschläge sowohl die Sicht behindern als auch den Nährboden für Bakterien bilden können, die zu Augeninfektionen führen.

Es ist deshalb notwendig Kontaktlinsen wenigstens einmal täglich gründlich zu reinigen und zu desinfizieren. Dabei müssen insbesondere die abgelagerten Proteine, Lipide und Mucine so zersetzt werden, daß sie durch Spüllösungen entfernbar sind.

Die Reinigung sogenannter weicher Kontaktlinsen ist besonders kritisch, da das weiche hydrophile Material der Linse Reinigungsmittel absorbiert und sogar konzentriert und diese später bei der Auflage auf dem Auge wieder abgeben kann. Die Kontaktlinse kann dadurch beschädigt oder verformt werden und empfindliche Gewebeabschnitte der Hornhaut können gereizt oder sogar beschädigt werden.

Deshalb ist es im allgemeinen nicht möglich Reinigungsmittel, die bei harten Kontaktlinsen mit ihren wenig oder nicht absorbierenden Flächen einsetzbar sind auch zur Reinigung weicher Kontaktlinsen zu verwenden.

Aus der DE-OS 24 17 844 ist ein Verfahren zum Reinigen weicher Kontaktlinsen bekannt, bei dem diese in eine praktisch isotonische wässrige Lösung gelegt werden, die eine wirksame Protease-Menge enthält. Als Protease wird vorwiegend das Enzym Papain verwendet. Hier erfolgt also die Auflösung der störenden Ablagerungen auf enzymatischen Wege.

Die DE-OS 28 54 278 schlägt vor die Lösungswirkung einer Protease enthaltenden Reinigungsmittels durch Zusatz eines nichtionogenen Netzmittels zu verbessern. Dabei soll das Reinigungsmittel in Form von Pulver, Tabletten oder flüssigem Konzentrat vorliegen, das vom Verbraucher in Wasser gelöst bzw. mit Wasser verdünnt wird.

Aus der DE-PS 24 43 147 ist ein Verfahren zur Reinigung von Kunststoff-Kontaktlinsen bekannt, bei dem die zu reinigende Linse zunächst mit einer basischen wässrigen Lösung einer aktiven Sauerstoff abgebenden Perverbindung behandelt, dann in eine saure wässrige Lösung einer aktiven Sauerstoff abgebenden Perverbindung eingebracht und schließlich nach Entfernen aus dieser Lösung mit einem nichtionischen Detergens behandelt und abschließend mit Wasser abgespült wird.

Dieses Verfahren ist recht umständlich, insbesondere da es notwendig ist drei fertige Lösungen zu verwenden. Es hat aber den Vorteil, daß es für weiche und harte Kontaktlinsen anwendbar ist, wenn auch mit Unterschieden im Verfahrensablauf insbes. bezüglich Zeit und Temperatur.

Es ist nun die Aufgabe der vorliegenden Erfindung ein Reinigungs- und Desinfektionssystem für harte und weiche Kontaktlinsen zu schaffen, das bei einfacher Handhabung Kontaktlinsenablagerungen sicher und schnell entfernt, und das für alle Arten von Kontaktlinsen gut verträglich und zudem reizfrei für das Auge des Kontaktlinsenträgers ist.

Diese Aufgabe wird durch ein Reinigungs- und Desinfektionssystem nach den Merkmalen des Anspruchs 1 gelöst.

Die Ansprüche 2 und 3 geben Stoffe an, die besonders vorteilhaft als Reduktionsmittel in Tablette B Verwendung finden.

Eine beispielsweise Dosierung der verwendeten Substanzen ist in den Ansprüchen 4 und 5 angegeben. Anspruch 6 gibt eine vorteilhafte Ausbildung des Reinigungsbehälters an, während Anspruch 7 einen beispielsweisen Ablauf des Reinigungsverfahrens angibt.

Bei dem Reinigungssystem nach der Erfindung werden die Reinigungslösungen in jedem Anwendungsfall frisch hergestellt, wobei durch die Verwendung von Tabletten und der fertigen Kochsalzlösung diese Herstellung leicht und einfach ist und zu Lösungen exakt gleicher Konzentration führt.

Die Verwendung von Tabletten bringt den weiteren Vorteil mit sich, daß das Reinigungssystem einfach und sicher zu transportieren, stets optimal wirksam und zudem sparsam im Verbrauch ist.

Die Tablette A dient entsprechend dem in Anspruch 4 wiedergegebenen Reinigungs- und Desinfektionsverfahren zur Herstellung der ersten Lösung, in die die verschmutzte Kontaktlinse gegeben wird. Das in dieser Tablette enthaltene Harnstoffperoxohydrat kann als "festes Wasserstoffperoxid" bezeichnet werden, das sich durch eine weit bessere Haltbarkeit als fertige Lösungen auszeichnet.

Beim Lösen der Tablette A in der Kochsalzlösung C entsteht eine Lösung, die Proteine, Lipide und Mucine oxidativ zersetzt und die durch ihren Gehalt an Harnstoff zusätzlich eine spezifische Ablösung von Proteeinkomponenten vom Kontaktlinsenmaterial bewirkt.

Die Kontaktlinse wird nach dem beschriebenen ersten Reinigungsvorgang in eine zweite Lösung eingegeben, die durch Einbringen einer Tablette B in Kochsalzlösung C entsteht. Die in der Ausbildung der Tablette B nach Anspruch 3 enthaltene L (+) Ascorbinsäure (Vitamin C) bewirkt eine schnelle reduktive Zersetzung von restlichem Wasserstoffperoxid. Als relativ kleines Molekül dringt L (+) Ascorbinsäure dabei in das Kontaktlinsenmaterial ein und zerstört restliches Wasserstoffperoxid. Diese Wirkung von L (+) Ascorbinsäure wird dadurch unterstützt, daß sich beim Auflösen des Natriumbicarbonat-Anteils Kohlendioxid entwickelt, wobei die Kontaktlinse schonend in der Lösung bewegt wird.

Derselbe Reduktionsprozeß wird auch bei Verwendung einer Tablette B in der Ausbildung nach Anspruch 2 erreicht, wobei allerdings kein "Brause-Effekt" erzielt wird. In der zweiten Lösung B/C wird also restliches Wasserstoffperoxid durch Reduktion so restlos zersetzt, daß spätere Reizungen des Auges ausgeschlossen sind. Diese Zersetzung erfolgt auch im Kontaktlinsenmaterial selbst, was insbesondere bei weichen Kontaktlinsen von besonderer Wichtigkeit ist.

- 4 -

Durch das anschließende Lagern der Kontaktlinse in der Kochsalzlösung C werden die zersetzten Ablagerungssubstanzen vollends abgelöst und die Linse wird äquilibiert.

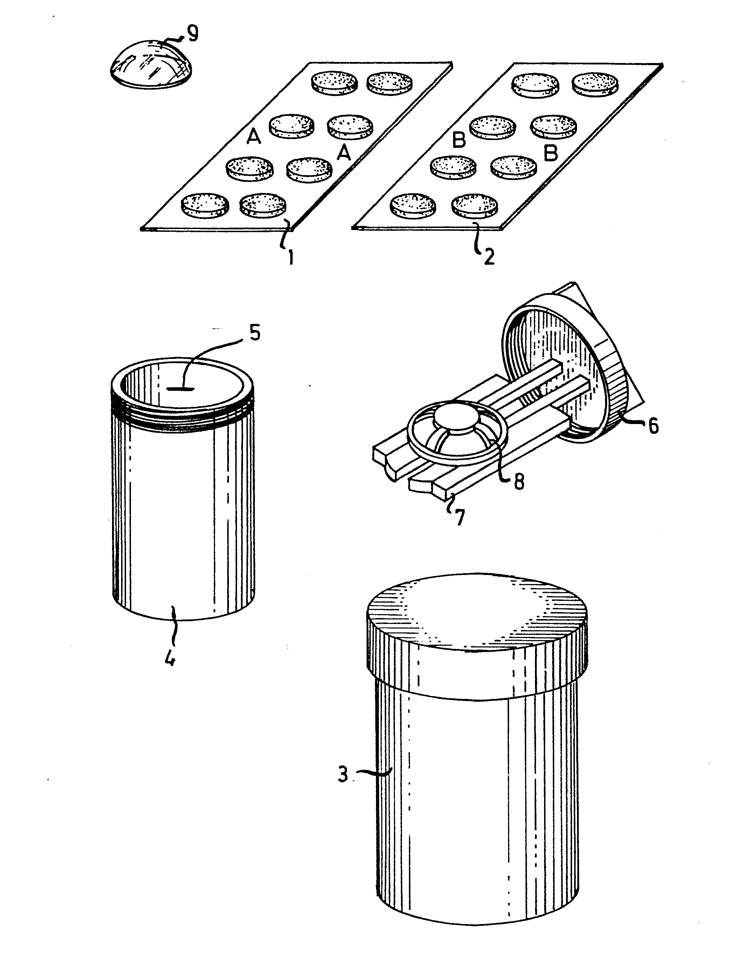

Zur weiteren Verdeutlichung der Erfindung wird auf die einzige Figur der beigefügten Zeichnung Bezug genommen, die ein Ausführungsbeispiel eines Reinigungs- und Desinfektions-Sets zeigt.

In der Figur ist mit 1 ein "Brief" bezeichnet, der einzeln verpackt in Alu/PE-Folie eine Anzahl von Tabletten A enthält. Jede Tablette besteht aus 1 Gramm Harnstoffperoxohydrat. Im "Brief" 2 sind Tabletten B einzeln verpackt. Jede Tablette besteht entweder aus einer Mischung von 0,1 bis 0,5 Gramm L (+) Ascorbinsäure (Vitamin C) und 0,1 bis 0,5 Gramm Natriumbicarbonat oder aus 0,1 bis 0,6 Gramm Natriumascorbat. Im Behälter 3 ist die Lösung C enthalten, die aus einer konservierten gepufferten 0,9%-igen Kochsalzlösung besteht.

Mit 4 ist ein Behälter bezeichnet, der bis zum Füllstrich 5 10 ml faßt. Zu diesem Behälter gehört ein Deckel 6, der einen Fortsatz 7 trägt, der mit einem Halter 8 zur Aufnahme der zu reinigenden Kontaktlinse versehen ist.

Zum Reinigen wird die mit 9 bezeichnete Kontaktlinse in den Halter 8 gegeben. Danach wird eine Tablette A in dem Behälter 4 gegeben und dieser wird bis zum Füllstrich 5 mit Lösung C gefüllt. Nach Aufsetzen des Deckels 6 bleibt die Linse 9 ca. 15 Minuten im Behälter 4, der gelegentlich geschüttelt wird.

Danach wird der Deckel 6 abgenommen und die Lösung A/C ausgegoßen. Als nächstes wird der Behälter 4 bis zum Füllstrich 5 mit Lösung C gefüllt, eine Tablette B hinzugegeben und der Deckel 6 aufgelegt, ohne daß bei Verwendung einer Natriumbicarbonat enthaltenden Tablette der Behälter 5 dadurch geschlossen wird. Die Linse 9 im Halter 8 bleibt ca. 5 Minuten in der Lösung B/C.

Diese wird danach ausgegossen und der Behälter 4 wird bis zum Füllstrich 5 mit Lösung C gefüllt. Nach Anschrauben des Deckels 6 bleibt

**0142623**

die Linse ca. 5 Minuten in der Lösung C.

Nach Abschluß dieser Schritte ist die Linse 9 gereinigt und desinfiziert und kann nach Entnahme aus dem Halter 8 wieder in das Auge eingesetzt werden.

Es ist auch möglich die Linse 9 nach Ausgießen der Lösung C im Halter 8 zu belassen und wieder in den mit einer Aufbewahrungslösung gefüllten Behälter 4 einzugeben.

Wie diese Darstellung zeigt ist der Reinigungs- und Desinfizierungsvorgang einfach und schnell durchzuführen. Er führt zu einer einwandfrei behandelten Kontaktlinse, die in das Auge eingesetzt werden kann, ohne daß Reizungen hervorgerufen werden.

Patentansprüche:

1. Reinigungs- und Desinfektionssystem für harte und weiche Kontaktlinsen, gekennzeichnet durch folgende Zusammensetzung

a) eine Tablette A, bestehend aus einer vorbestimmten Menge Hornstoffperoxohydrat,

b) eine Tablette B, bestehend aus einer vorbestimmten Menge eines
Reduktionsmittels,

c) einer konservierten, gepufferten Kochsalzlösung C vorbestimmter
Konzentration,

d) einem mit Füllstrich (5) versehenen Behälter (4) zur Aufnahme der
zu reinigenden Kontaktlinse (9) in aufeinanderfolgend

   - einer Lösung einer Tablette A in Kochsalzlösung C, aufgefüllt
     bis zum Füllstrich,

   - einer Lösung einer Tablette B in Kochsalzlösung C, aufgefüllt
     bis zum Füllstrich,

   - reiner Kochsalzlösung C

2. Reinigungs- und Desinfektionssystem nach Anspruch 1, dadurch gekennzeichnet, daß die Tablette B aus Natriumascorbat besteht.

3. Reinigungs- und Desinfektionssystem nach Anspruch 1, dadurch gekennzeichnet, daß die Tablette B aus einer Mischung von L (+) Ascorbinsäure und Natriumcarbonat besteht.

4. Reinigungs- und Desinfektionssystem nach Anspruch 2, dadurch gekennzeichnet, daß
die Tablette A aus 1 Gramm Harnstoffperoxohydrat,
die Tablette B aus 0,1-0,6 Gramm Natriumascorbat besteht,
die Kochsalzlösung C 0,9%-ig ist, und
der Behälter (4) bis zum Füllstrich (5) 10 ml faßt.

5. Reinigungs- und Desinfektionssystem nach Anspruch 3, dadurch gekennzeichnet, daß
die Tablette A aus 1 Gramm Harnstoffperoxohydrat,
die Tablette B aus einer Mischung von 0,1 bis 0,5 Gramm L (+) Ascorbinsäure (Vitamin C) und 0,1 bis 0,5 Gramm Natriumbicarbonat besteht,

die Kochsalzlösung C 0,9%-ig ist, und

der Behälter (4) bis zum Füllstrich (5) 10 ml faßt.

6. Reinigungs- und Desinfektionssystem nach Anspruch 1 bis 5, dadurch gekennzeichnet, daß der Behälter (4) mit einem Halter (8) zur Aufnahme der zu reinigenden Kontaktlinse (9) ausgestattet ist.

7. Verfahren zum Reinigen und Desinfizieren von harten und weichen Kontaktlinsen unter Verwendung des Systems der Ansprüche 1-6, dadurch gekennzeichnet, daß

a) eine Tablette A in 10 ml der 0,9%-igen Kochsalzlösung C gelöst und die Linse 10-20 Minuten in dieser Lösung gelagert wird, anschließend

b) eine Tablette B in 10 ml der 0,9%-igen Kochsalzlösung C eingebracht und die Linse ca. 5 Minuten in dieser Lösung gelagert wird; und

c) anschließend die Linse mindestens 5 Minuten in der 0,9%-igen Kochsalzlösung gelagert wird.

**Europäisches Patentamt**

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | US-A-3 873 696 (K.J. RANDERI) <br> * Spalte 1, Zeilen 63-68; Spalte 2, Zeilen 1-15; Spalte 3, Zeilen 17-18; Spalte 4, Zeilen 1,2 * | 1,7 | A 61 L 2/18 <br> G 02 C 13/00 |
| D,Y | FR-A-2 247 327 (FLOW PHARMACEUTICALS) <br> * Seite 5, Zeilen 26-30; Ansprüche 1-4 * | 1,7 | |
| Y | FR-A-2 400 906 (H.C.A. EVERS) <br> * Beispiele 1-3; Ansprüche 1,9 * | 1,7 | |
| A | FR-A-2 134 666 (ALLERGAN) <br><br> * Seite 3, Zeilen 6-14 * | | |

RECHERCHIERTE SACHGEBIETE (Int. Cl.4)

A 61 L
G 02 C

Der vorliegende Recherchenbericht wurde für alle Patentansprüche erstellt.

| Recherchenort | Abschlußdatum der Recherche | Prüfer |
|---|---|---|
| DEN HAAG | 27-11-1984 | PELTRE CHR. |